# EUROPEAN PATENT APPLICATION

(11) **EP 2 103 311 A1**
(43) Date of publication of application: **23.09.2009**
(21) Application number: 08005320.0
(22) Date of filing: 20.03.2008
(51) Int. Cl.: A61K 39/395, A61P 7/02

(54) **The calcium sensor STIM1 is essential for pathological thrombus formation**

(71) Applicant: CSL Behring GmbH, 35041 Marburg (DE); Julius-Maximilians-Universität Würzburg, 97070 Würzburg (DE)
(72) Inventor: Braun, Attila, 97070 Würzburg (DE); Kleinschnitz, Christoph, 97230 Estenfeld (DE); Nieswandt, Bernhard, 97246 Eibelstadt (DE); Stoll, Guido, 97222 Rimpar (DE); Varga-Szabó, Dávid, 42119 Wuppertal (DE)

(57) **Abstract**

The present invention relates to a pharmaceutical composition comprising an inhibitor of stromal interaction molecule 1 (STIM1) or an inhibitor of an activator of STIM1 and optionally a pharmaceutically active carrier, excipient or diluent. The invention further relates to an inhibitor of stromal interaction molecule 1 (STIM1) or an inhibitor of an activator of STIM1 for treating or preventing a disorder related to venous or arterial thrombus formation.

## Description

The present invention relates to a pharmaceutical composition comprising an inhibitor of stromal interaction molecule 1 (STIM1) or an inhibitor of an activator of STIM1 and optionally a pharmaceutically active carrier, excipient or diluent. The invention further relates to an inhibitor of stromal interaction molecule 1 (STIM1) or an inhibitor of an activator of STIM1 for treating or preventing a disorder related to venous or arterial thrombus formation.

In this specification, a number of documents are cited. The disclosure content of these documents including manufacturer's manuals is herewith incorporated by reference in its entirety.

At sites of vessel wall injury, components of the extracellular matrix (ECM) are exposed to the flowing blood which triggers sudden platelet activation and platelet plug formation, followed by coagulant activity and the formation of fibrin-containing thrombi that occlude the site of injury. These events are crucial to prevent posttraumatic blood loss but they are also a major pathological mechanism underlying precipitate diseases such as myocardial infarction and stroke (George, J.N. 2000, Lancet 355:1531-1539; Ruggeri, Z.M. 2000, Nat Med 8:1227-1234). Inhibition of platelet function is an important strategy for prevention and treatment of myocardial infarction (Bhatt, D.L. and Topol,E.J. 2003, Nat. Rev. Drug Discov. 2:15-28) and possibly stroke (Bhatt, D.L. and Topol,E.J. 2003, Nat. Rev. Drug Discov. 2:15-28; Kleinschnitz, C. et al., 2007, Circulation 115:2323-2330). Platelet activation is triggered by subendothelial collagens, thromboxane A₂ (TxA₂) and ADP released from activated platelets, and thrombin generated by the coagulation cascade (Sachs, U.J. and Nieswandt,B. 2007, Circ. Res. 100:979-991). Although these agonists trigger different signaling pathways, all activate phospholipase (PL) Cs, leading to production of diacylglycerol (DAG) and inositol 1,4,5-triphosphate (IP₃). IP₃ induces release of Ca²⁺ from the ER, which is thought to trigger the influx of extracellular Ca²⁺ by a mechanism known as *store-operated* Ca²⁺ *entry* (SOCE) (Berridge, M.J. et al., 2003, Nat. Rev. Mol. Cell Biol. 4:517-529; Rosado, J.A. et al., 2005, J. Cell Physiol 205:262-269; Feske, S., 2007, Nat. Rev. Immunol. 7:690-702). In addition, DAG and some of its metabolites have been shown to induce *non-store operated Ca²⁺ entry* (non-SOCE) (Bird, G.S. et al., 2004, Mol. Med. 4:291-301).

Stromal interaction molecule 1 (STIM1) is an ER-resident protein necessary for detection of ER Ca²⁺-depletion and activation of *store-operated Ca²⁺* (SOC) channels in Jurkat T cells ( Roos, J. et al., 2005, J. Cell Biol. 169:435-445; Liou, J. et al., 2005, Curr. Biol. 15:1235-1241; Zhang, S.L. et al., 2005, Nature 437:902-905; Peinelt, C. et al., 2006, Nat. Cell Biol. 8:771-773) and mast cells (Baba, Y. et al., 2007, Nat. Immunol). In human T cells, the four transmembrane domain protein Orai1 (CRACM) appears to be the predominant SOC channel (Feske, S. et al., 2006, Nature 441:179-185; Vig, M. et al., 2006, Science 312:1220-1223), but the C-terminal region of STIM1 also interacts with other SOC channel candidates such as *transient receptor potential channels* TRPCs 1, 2 and 4 (Huang, G.N. et al., 2006, Nat. Cell Biol. 8:1003-1010). In platelets, STIM1 is expressed at high levels (Grosse, J. et al., 2007, J. Clin. Invest 117:3540-3550) and may contribute to SOCE by interacting with TRPC1 (Lopez, J. et al., 2006, J. Biol. Chem. 281:28254-28264). It has recently been reported that mice expressing an activating EF-hand mutant of STIM1 have elevated [Ca²⁺]ᵢ levels in platelets, macrothrombocytopenia and a bleeding disorder, indicating a role for STIM1-dependent SOCE in platelet function (Grosse, J. et al., 2007, J. Clin. Invest 117:3540-3550). The importance of SOCE for platelet activation, hemostasis, and thrombosis, however, remains unknown, and the mechanisms underlying the process are not defined.

Despite the fact that thrombus formation leads to some of the most frequently occurring diseases in humans und despite extensive basic and clinical research that has been carried out over decades, medicaments that have been registered and are presently available for patients are unsatisfactory for a variety of reasons. Thus, all anti-coagulants presently used in clinics are associated with an increased risk of serious bleeding. These include heparins, cumarins, direct thrombin inhibitors such as hirudin, as well as aspirin, P2Y₁₂ inhibitors such as clopidogrel and GPIIb/IIIa inhibitors such as abciximab (ReoPro). On the other hand, many anticoagulants / antiplatelet agents have additional undesired effects such as the induction of thrombocytopenia. This is best described for heparin (heparin-induced thrombocytopenia, HIT) (Hassan, Y. et al., 2007, J. Clin. Pharm. Ther. 32:535-544) or GPIIb/IIIa blockers (abciximab, ReoPro) (Höchtl, T., 2007, J. Thromb. Thrombolysis. 24:59-64.). For other prominent inhibitors such as aspirin or the P2Y₁₂ inhibitor clopidogrel, many patients have been described as low or non-responders (Papthanasiou et al., 2007, Hellenic J. Cardiol. 48:352-363). Thus, the technical problem underlying the present invention was to provide novel approaches for successfully targeting diseases based on thrombus formation that form the basis or may allow the development of more satisfactory medicaments for the treatment and/or prevention of the mentioned diseases.

Accordingly, the present invention relates to a pharmaceutical composition comprising an inhibitor of stromal interaction molecule 1 (STIM1) or an inhibitor of an activator of STIM1 and optionally a pharmaceutically active carrier, excipient and/or diluent.

The term "pharmaceutical composition" as employed herein comprises at least one such as at least two, e.g. at least three, in further embodiments at least four such as at last five of the mentioned inhibitors. The invention also envisages mixtures of inhibitors of stromal interaction molecule 1 (STIM1) and inhibitors of an activator of STIM1.

The composition may be in solid, liquid or gaseous form and may be, inter alia, in a form of (a) powder(s), (a) tablet(s), (a) solution(s) or (an) aerosol(s).

It is preferred that said pharmaceutical composition comprises a pharmaceutically acceptable carrier, excipient and/or diluent. Examples of suitable pharmaceutical carriers, excipients and/or diluents are well known in the art and include phosphate buffered saline solutions, water, emulsions, such as oil/water emulsions, various types of wetting agents, sterile solutions etc. Compositions comprising such carriers can be formulated by well known conventional methods. These pharmaceutical compositions can be administered to the subject at a suitable dose. Administration of the suitable compositions may be effected by different ways, e.g., by intravenous, intraperitoneal, subcutaneous, intramuscular, topical, intradermal, intranasal or intrabronchial administration. It is particularly preferred that said administration is carried out by injection and/or delivery, e.g., to a site in the bloodstream.. The compositions of the invention may also be administered directly to the target site, e.g., by biolistic delivery to an external or internal target site, like the brain. The dosage regimen will be determined by the attending physician and clinical factors. As is well known in the medical arts, dosages for any one patient depends upon many factors, including the patient's size, body surface area, age, the particular compound to be administered, sex, time and route of administration, general health, and other drugs being administered concurrently. Proteinaceous pharmaceutically active matter may be present in amounts between 1 ng and 10 mg/kg body weight per dose; however, doses below or above this exemplary range are envisioned, especially considering the aforementioned factors. If the regimen is a continuous infusion, it should also be in the range of 1 µg to 10 mg units per kilogram of body weight per minute.

Progress can be monitored by periodic assessment. The compositions of the invention may be administered locally or systemically. Preparations for parenteral administration include sterile aqueous or non-aqueous solutions, suspensions, and emulsions. Examples of non-aqueous solvents are propylene glycol, polyethylene glycol, vegetable oils such as olive oil, and injectable organic esters such as ethyl oleate. Aqueous carriers include water, alcoholic/aqueous solutions, emulsions or suspensions, including saline and buffered media. Parenteral vehicles include sodium chloride solution, Ringer's dextrose, dextrose and sodium chloride, lactated Ringer's, or fixed oils. Intravenous vehicles include fluid and nutrient replenishers, electrolyte replenishers (such as those based on Ringer's dextrose), and the like. Preservatives and other additives may also be present such as, for example, antimicrobials, anti-oxidants, chelating agents, and inert gases and the like. It is particularly preferred that said pharmaceutical composition comprises further agents known in the art to antagonize thrombus formation or to reduce thrombus size. Since the pharmaceutical preparation of the present invention relies on the above mentioned inhibitors, it is preferred that those mentioned further agents are only used as a supplement, i.e. at a reduced dose as compared to the recommended dose when used as the only drug, so as to e.g. reduce side effects conferred by the further agents. Conventional excipients include binding agents, fillers, lubricants and wetting agents.

The term "inhibitor of stromal interaction molecule 1 (STIM1)" refers to an inhibitor that reduces the biological function of STIM1 to at least 50%, preferably to at least 75%, more preferred to at least 90% and even more preferred to at least 95% such as at least 98% or even at least 99%. Biological function denotes in particular any known biological function of STIM1 or any combination thereof including functions elucidated in accordance with the present invention. Examples of said biological function is the binding capacity of STIM1 to SOC channel candidates mentioned herein above such as transient receptor potential channels (TRPCs), the activation of store-operated Ca²⁺ (SOC) channels including members of the Orai family of channels, in particular Orai1, the capability to adhere to collagen fibers, in particular under intermediate (e.g. 1000s⁻¹) or high shear conditions (e.g. 1,700 s⁻¹), the capability to efficiently degranulate, the contribution to the formation of thrombus formation such as three dimensional thrombus formation, in particular pathologic occlusive thrombus formation (platelet-rich thrombi), the contribution to normal hemostasis and the contribution to platelet activation.

The term "inhibitor of an activator of STIM1" refers to inhibitors that do not directly interact with STIM1 but with molecules that directly or indirectly activate one or more of the biological functions of STIM1 and preferably one or more of those functions referred to above or elsewhere in this specification. These include inhibitors of IP3-dependent Ca²⁺ store-release, inhibitors of STIM1 associated proteins involved in intracellular motility of STIM1 or SOC channel activation. The inhibition values referred to above for inhibitors of STIM1 *mutatis mutandis* apply to inhibitors of an activator of STIM1.

Stromal interaction molecule 1 (STIM1) has been identified as the long-sought calcium sensor that connects intracellular Ca²⁺ store-depletion to the activation of plasma membrane SOC channels in immune cells. Although SOCE was considered in the art to be a major pathway of Ca²⁺ entry in virtually all non-excitable cells, this has only been directly shown for T cells (Roos, J. et al., 2005, J. Cell Biol. 169:435-445; Zhang, S.L. et al., 2005, Nature 437:902-905) and mast cells (Baba, Y. et al., 2007, Nat. Immunol). In accordance with the present invention, it was now surprisingly shown that STIM1 is required for efficient platelet activation and thrombus formation. In the course of the present invention, mice deficient in STIM1 were generated and their platelets analyzed. It was found that Ca²⁺ responses to all major agonsists were defective resulting in impaired thrombus formation under flow in vitro and protection from arterial thrombosis and ischemic brain infaction in vivo. The ability of *STIM1*^{*-*/*-*} platelets to stabilize large thrombi under flow is impaired both *in vitro* and *in vivo,* demonstrating an important function of STIM1-dependent SOCE in thrombus formation under conditions of elevated shear. Despite this defect, *STIM1*^{*-*/*-*} platelets aggregate *in vitro* and can contribute to hemostasis *in vivo* making STIM1-dependent SOCE an attractive target for the prevention or treatment of acute ischemic events.

Although STIM1 is highly expressed in platelets (Grosse, J. et al., 2007, J. Clin. Invest 117:3540-3550), the significance of SOCE for platelet function has been completely unknown because non-SOCE pathways have been described to exist in these cells (Hassock, S.R. et al., 2002, Blood 100:2801-2811). The present inventors found largely defective Ca²⁺ responses to all major agonists in *STIM1*^{*-*/-}platelets, clearly establishing SOCE as the major route of Ca²⁺ entry in those cells and STIM1 as an essential mediator of this process. The residual Ca²⁺ influx detected in *STIM1*^{*-*/*-*} platelets suggests that other molecules may regulate SOC influx, but only to a minor extent. One candidate molecule is STIM2, which was originally reported to be an inhibitor of STIM1 (Soboloff, J. et al., 2006, Curr. Biol. 16:1465-1470) but later shown by the same group to activate CRAC channels (Parvez, S. et al., 2007, FASEB J). Alternatively, the residual Ca²⁺ entry could be mediated by store-independent mechanisms as DAG and some of its metabolites have been shown to induce non-SOCE (Bird, G.S. et al., 2004, Mol. Med. 4:291-301). Members of the TrpC family have been suggested as candidates mediating both, SOCE and non-SOCE (Rosado, J.A. et al., 2005, J. Cell Physiol 205:262-269; Lopez, J. et al., 2006, J. Biol. Chem. 281:28254-28264; Hassock, S.R. et al., 2002, Blood 100:2801-2811).

Beside the severely impaired SOCE already reduced Ca²⁺ release from intracellular stores upon agonist induced platelet activation was observed, which turned out to be the result of the lower filling state of the ER, as shown by passively emptying the stores with the SERCA inhibitor thapsigargin. Although the role of STIM1 in regulating the filling state of the ER is unknown, an explanation could be that in STIM1^{-/-} platelets the defective SOC channels can not fulfil one of their proposed major roles, namely to maintain the calcium content of the intracellular stores. Alternatively, STIM1 could interact with the IP₃ receptors or SERCA pumps in the ER, thereby influencing their function directly and resulting in impaired calcium release from the endoplasmic reticulum.

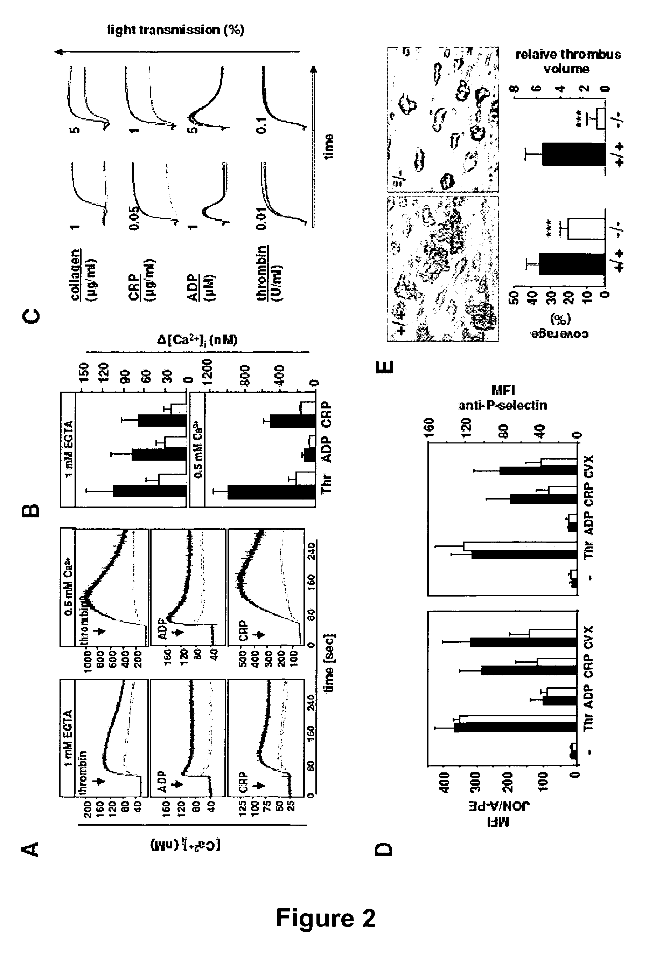

As demonstrated by the appended examples, although STIM-deficiency reduced Ca²⁺ entry in platelets in response to all agonists tested, it did not impair Gq/PLCβ-triggered integrin allbβ3 activation or release of granule content in the absence of flow (Fig. 2). This shows that SOCE is not essential for these processes when the agonist can act on the cells at constant concentrations for a prolonged period of time. In contrast, GPVI/PLCγ2-induced cellular activation was impaired under these experimental conditions, even at very high agonist concentrations (Fig. 2c). This could be related to the fact that GPVI and GPCRs activate different phospholipase C isoforms in platelets. GPVI ligation triggers tyrosine phosphorylation cascades downstream of the receptor-associated *immunoreceptor tyrosine activation motif* (ITAM) culminating in the activation of phospholipase (PL)Cγ2(29) whereas soluble agonists such as thrombin, ADP and TxA₂ stimulate receptors that couple to heterotrimeric G proteins (Gq) and lead to activation of PLCβ(30). The Ca²⁺ measurements show that store release and subsequent SOC influx occur significantly faster upon Gq/PLCβ stimulation compared to GPVI/PLCγ2 stimulation (Fig. 2a), suggesting different kinetics of IP₃ production between these two pathways which could influence subsequent events.

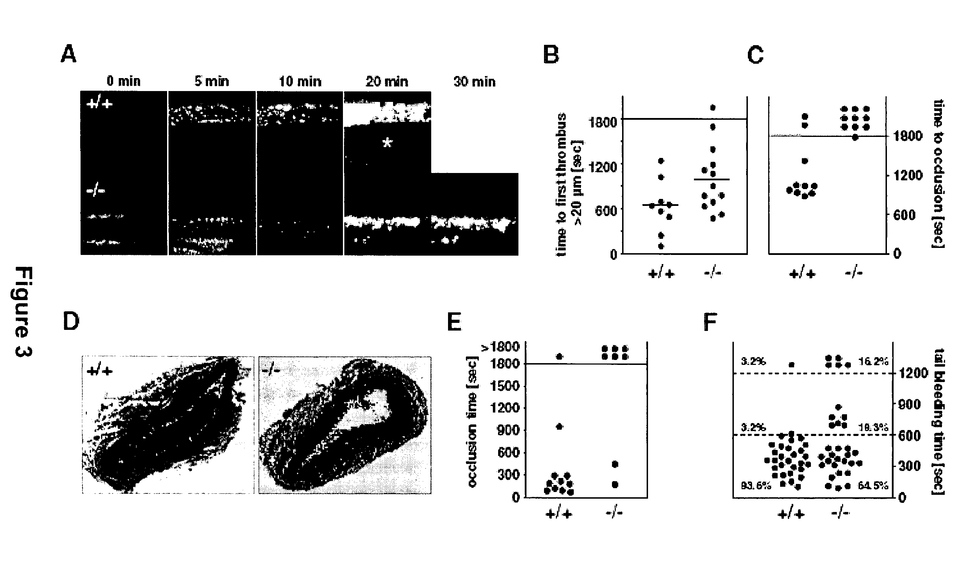

The rather mild activation deficits seen in *STIM1*^{*-*/*-*} platelets in the absence of flow translated into severely defective formation of stable three-dimensional thrombi under conditions of medium and high shear (Fig. 3). This suggests that STIM1-dependent SOCE is particularly important under conditions where agonist potency becomes limited due to rapid dilution and various stimuli have to be integrated to produce an appropriate cellular response.

Taken together, the results presented here establish STIM1 as an essential mediator of platelet activation that is of pivotal importance during arterial thrombosis and ischemic brain infarction. Thus, the above findings allow for the preparation of pharmaceutical compositions on the basis of inhibitors of stromal interaction molecule 1 (STIM1) or inhibitors of an activator of STIM1. The inhibitors will be useful as medicaments for a variety of diseases relating to thrombus formation which will be discussed in more detail herein below. Most importantly, since the inhibitor to STIM1 is not expected to have any effect on hemostasis, the envisaged drugs will not only be highly effective but also safe antithrombotics.

Further, the invention relates to an inhibitor of stromal interaction molecule 1 (STIM1) or an inhibitor of an activator of STIM1 for treating or preventing a disorder related to venous or arterial thrombus formation. Alternatively, the mentioned inhibitor may be used as a lead compound for the development of a drug for treating or preventing a disorder related to venous or arterial thrombus formation. Those lead compounds will also allow for the development of novel, highly effective, yet safe antithrombotics. In the development of those drugs, the following developments are considered: (i) modified site of action, spectrum of activity, organ specificity, and/or (ii) improved potency, and/or (iii) decreased toxicity (improved therapeutic index), and/or (iv) decreased side effects, and/or (v) modified onset of therapeutic action, duration of effect, and/or (vi) modified pharmacokinetic parameters (resorption, distribution, metabolism and excretion), and/or (vii) modified physico-chemical parameters (solubility, hygroscopicity, color, taste, odor, stability, state), and/or (viii) improved general specificity, organ/tissue specificity, and/or (ix) optimized application form and route by (i) esterification of carboxyl groups, or (ii) esterification of hydroxyl groups with carboxylic acids, or (iii) esterification of hydroxyl groups to, e.g. phosphates, pyrophosphates or sulfates or hemi-succinates, or (iv) formation of pharmaceutically acceptable salts, or (v) formation of pharmaceutically acceptable complexes, or (vi) synthesis of pharmacologically active polymers, or (vii) introduction of hydrophilic moieties, or (viii) introduction/exchange of substituents on aromates or side chains, change of substituent pattern, or (ix) modification by introduction of isosteric or bioisosteric moieties, or (x) synthesis of homologous compounds, or (xi) introduction of branched side chains, or (xii) conversion of alkyl substituents to cyclic analogues, or (xiii) derivatisation of hydroxyl group to ketales, acetales, or (xiv) N-acetylation to amides, phenylcarbamates, or (xv) synthesis of Mannich bases, imines, or (xvi) transformation of ketones or aldehydes to Schiff's bases, oximes, acetales, ketales, enolesters, oxazolidines, thiazolidines or combinations thereof.

The various steps recited above are generally known in the art. They include or rely on quantitative structure-action relationship (QSAR) analyses (Kubinyi, "Hausch-Analysis and Related Approaches", VCH Verlag, Weinheim, 1992), combinatorial biochemistry, classical chemistry and others (see, for example, Holzgrabe and Bechtold, Deutsche Apotheker Zeitung 140(8), 813-823, 2000).

In a preferred embodiment of the pharmaceutical composition or the inhibitor of the invention, the inhibitor is an antibody, an aptamer, an siRNA, an shRNA, or a further molecule effecting RNAi, a ribozyme, an antisense nucleic acid molecule or a small molecule.

The antibody in accordance with the present invention can be, for example, polyclonal or monoclonal. The term "antibody" also comprises derivatives or fragments thereof which still retain the binding specificity. Techniques for the production of antibodies are well known in the art and described, e.g. in Harlow and Lane "Antibodies, A Laboratory Manual", Cold Spring Harbor Laboratory Press, 1988 and Harlow and Lane "Using Antibodies: A Laboratory Manual" Cold Spring Harbor Laboratory Press, 1999.

The antibody also includes embodiments such as chimeric (human constant domain, non-human variable domain), single chain and humanized (human antibody with the exception of non-human CDRs) antibodies, as well as antibody fragments, like, inter alia, Fab fragments. Antibody fragments or derivatives further comprise F(ab')₂, Fv or scFv fragments; see, for example, Harlow and Lane (1988) and (1999), loc. cit.. Various procedures are known in the art and may be used for the production of such antibodies and/or fragments. Thus, the (antibody) derivatives can be produced by peptidomimetics. Further, techniques described for the production of single chain antibodies (see, inter alia, US Patent 4,946,778) can be adapted to produce single chain antibodies specific for polypeptide(s) and fusion proteins of this invention. Also, transgenic animals may be used to express humanized antibodies specific for polypeptides and fusion proteins of this invention. Most preferably, the antibody is a monoclonal antibody, such as a human or humanized antibody. For the preparation of monoclonal antibodies, any technique which provides antibodies produced by continuous cell line cultures can be used. Examples for such techniques include the hybridoma technique (Köhler and Milstein Nature 256 (1975), 495-497), the trioma technique, the human B-cell hybridoma technique (Kozbor, Immunology Today 4 (1983), 72) and the EBV-hybridoma technique to produce human monoclonal antibodies (Cole et al., Monoclonal Antibodies and Cancer Therapy, Alan R. Liss, Inc. (1985), 77-96). Surface plasmon resonance as employed in the BlAcore system can be used to increase the efficiency of phage antibodies which bind to an epitope of STIM1 of an activator of STIM1 (Schier, Human Antibodies Hybridomas 7 (1996), 97-105; Malmborg, J. Immunol. Methods 183 (1995), 7-13). It is also envisaged in the context of this invention that the term "antibody" comprises antibody constructs which may be expressed in cells, e.g. antibody constructs which may be transfected and/or transduced via, inter alia, viruses or plasmid vectors.

Aptamers are oligonucleic acid or peptide molecules that bind a specific target molecule. Aptamers are usually created by selecting them from a large random sequence pool, but natural aptamers also exist in riboswitches. Aptamers can be used for both basic research and clinical purposes as macromolecular drugs. Aptamers can be combined with ribozymes to self-cleave in the presence of their target molecule. These compound molecules have additional research, industrial and clinical applications (Osborne et. al. (1997), Current Opinion in Chemical Biology, 1:5-9; Stull & Szoka (1995), Pharmaceutical Research, 12, 4:465-483).

More specifically, aptamers can be classified as DNA or RNA aptamers or peptide aptamers. Whereas the former normally consist of (usually short) strands of oligonucleotides, the latter preferably consist of a short variable peptide domain, attached at both ends to a protein scaffold.

Nucleic acid aptamers are nucleic acid species that, as a rule, have been engineered through repeated rounds of in vitro selection or equivalently, SELEX (systematic evolution of ligands by exponential enrichment) to bind to various molecular targets such as small molecules, proteins, nucleic acids, and even cells, tissues and organisms.

Peptide aptamers usually are peptides or proteins that are designed to interfere with other protein interactions inside cells. They consist of a variable peptide loop attached at both ends to a protein scaffold. This double structural constraint greatly increases the binding affinity of the peptide aptamer to levels comparable to an antibody's (nanomolar range). The variable loop length is typically comprised of 10 to 20 amino acids, and the scaffold may be any protein which have good solubility properties. Currently, the bacterial protein Thioredoxin-A is the most used scaffold protein, the variable loop being inserted within the reducing active site, which is a - Cys-Gly-Pro-Cys- loop in the wild protein, the two cysteins lateral chains being able to form a disulfide bridge. Peptide aptamer selection can be made using different systems, but the most used is currently the yeast two-hybrid system.

Aptamers offer the utility for biotechnological and therapeutic applications as they offer molecular recognition properties that rival those of the commonly used biomolecules, in particular antibodies. In addition to their discriminate recognition, aptamers offer advantages over antibodies as they can be engineered completely in a test tube, are readily produced by chemical synthesis, possess desirable storage properties, and elicit little or no immunogenicity in therapeutic applications.

Non-modified aptamers are cleared rapidly from the bloodstream, with a half-life of minutes to hours, mainly due to nuclease degradation and clearance from the body by the kidneys, a result of the aptamer's inherently low molecular weight. Unmodified aptamer applications currently focus on treating transient conditions such as blood clotting, or treating organs such as the eye where local delivery is possible. This rapid clearance can be an advantage in applications such as in vivo diagnostic imaging. Several modifications, such as 2'-fluorine-substituted pyrimidines, polyethylene glycol (PEG) linkage, etc. are available to scientists with which the half-life of aptamers easily can be increased to the day or even week time scale.

The term "peptide" as used herein describes a group of molecules consisting of up to 30 amino acids, as well as the group of polypeptides, whereas "proteins"consist of more than 30 amino acids. Peptides and proteins may further form dimers, trimers and higher oligomers, i.e. consisting of more than one molecule which may be identical or non-identical. The corresponding higher order structures are, consequently, termed homo- or heterodimers, homo- or heterotrimers etc. The terms "peptide" and "protein" also refer to naturally modified peptides/proteins wherein the modification is effected e.g. by glycosylation, acetylation, phosphorylation and the like. Such modifications are well-known in the art.

For therapeutic uses, the RNA inactivation by antisense molecules or by ribozymes is implementable. Both classes of compounds can be synthesized chemically or produced in conjunction with a promoter by biological expression in vitro or even in vivo.

Small interfering RNA (siRNA), sometimes known as short interfering RNA or silencing RNA, are a class of 18 to 30, preferably 20 to 25, most preferred 21 to 23 or 21 nucleotide-long double-stranded RNA molecules that play a variety of roles in biology. Most notably, siRNA is involved in the RNA interference (RNAi) pathway where the siRNA interferes with the expression of a specific gene. In addition to their role in the RNAi pathway, siRNAs also act in RNAi-related pathways, e.g. as an antiviral mechanism or in shaping the chromatin structure of a genome.

Natural siRNAs have a well defined structure: a short double-strand of RNA (dsRNA) with 2-nt 3' overhangs on either end. Each strand has a 5' phosphate group and a 3' hydroxyl (-OH) group. This structure is the result of processing by dicer, an enzyme that converts either long dsRNAs or small hairpin RNAs into siRNAs. SiRNAs can also be exogenously (artificially) introduced into cells to bring about the specific knockdown of a gene of interest. Essentially any gene of which the sequence is known can thus be targeted based on sequence complementarity with an appropriately tailored siRNA.

The double-stranded RNA molecule or a metabolic processing product thereof is capable of mediating target-specific nucleic acid modifications, particularly RNA interference and/or DNA methylation. Preferably at least one RNA strand has a 5'-and/or 3'-overhang. Preferably, one end of the double-strand has a 3'-overhang from 1-5 nucleotides, more preferably from 1-3 nucleotides and most preferably 2 nucleotides. The other end may be blunt-ended or has up to 6 nucleotides 3'-overhang. In general, any RNA molecule suitable to act as siRNA is envisioned in the present invention.

The most efficient silencing was so far obtained with siRNA duplexes composed of 21-nt sense and 21-nt antisense strands, paired in a manner to have a 2-nt 3'-overhang. The sequence of the 2-nt 3' overhang makes a small contribution to the specificity of target recognition restricted to the unpaired nucleotide adjacent to the first base pair (Elbashir et al. 2001). 2'-deoxynucleotides in the 3' overhangs are as efficient as ribonucleotides, but are often cheaper to synthesize and probably more nuclease resistant.

A short hairpin RNA (shRNA) is a sequence of RNA that makes a tight hairpin turn that can be used to silence gene expression via RNA interference. shRNA uses a vector introduced into cells and utilizes the U6 promoter to ensure that the shRNA is always expressed. This vector is usually passed on to daughter cells, allowing the gene silencing to be inherited. The shRNA hairpin structure is cleaved by the cellular machinery into siRNA, which is then bound to the RNA-induced silencing complex (RISC). This complex binds to and cleaves mRNAs which match the siRNA that is bound to it.

Si/shRNAs to be used in the present invention are preferably chemically synthesized using appropriately protected ribonucleoside phosphoramidites and a conventional DNA/RNA synthesizer. Suppliers of RNA synthesis reagents are Proligo (Hamburg, Germany), Dharmacon Research (Lafayette, CO, USA), Pierce Chemical (part of Perbio Science, Rockford, IL , USA), Glen Research (Sterling, VA, USA), ChemGenes (Ashland, MA, USA), and Cruachem (Glasgow, UK). Most conveniently, siRNAs or shRNAs are obtained from commercial RNA oligo synthesis suppliers, which sell RNA-synthesis products of different quality and costs. In general, the RNAs applicable in the present invention are not too difficult to synthesize and are readily provided in a quality suitable for RNAi.

Further molecules effecting RNAi include, for example, microRNAs (miRNA). Said RNA species are single-stranded RNA molecules which as endogenous RNA molecules regulate gene expression. Upon binding to a complementary mRNA transcript triggers the degradation of said mRNA transcript through a process similar to RNA interference. Accordingly, miRNAs may be employed to regulate the expression of SMT1.

A ribozyme (from ribonucleic acid enzyme, also called RNA enzyme or catalytic RNA) is an RNA molecule that catalyzes a chemical reaction. Many natural ribozymes catalyze either their own cleavage or the cleavage of other RNAs, but they have also been found to catalyze the aminotransferase activity of the ribosome.

Examples of well-characterized small self-cleaving RNAs are the hammerhead, hairpin, hepatitis delta virus, and in vitro-selected lead-dependent ribozymes. The organization of these small catalysts is contrasted to that of larger ribozymes, such as the group I intron.

The principle of catalytic self-cleavage has become well established in the last 10 years. The hammerhead ribozymes are characterized best among the RNA molecules with ribozyme activity. Since it was shown that hammerhead structures can be integrated into heterologous RNA sequences and that ribozyme activity can thereby be transferred to these molecules, it appears that catalytic antisense sequences for almost any target sequence can be created, provided the target sequence contains a potential matching cleavage site.

The basic principle of constructing hammerhead ribozymes is as follows: An interesting region of the RNA, which contains the GUC (or CUC) triplet, is selected. Two oligonucleotide strands, each usually with 6 to 8 nucleotides, are taken and the catalytic hammerhead sequence is inserted between them. Molecules of this type were synthesized for numerous target sequences. They showed catalytic activity in vitro and in some cases also in vivo. The best results are usually obtained with short ribozymes and target sequences.

A recent development, also useful in accordance with the present invention, is the combination of an aptamer recognizing a small compound with a hammerhead ribozyme. The conformational change induced in the aptamer upon binding the target molecule, is supposed to regulate the catalytic function of the ribozyme.

The term "antisense nucleic acid molecule" is known in the art and refers to a nucleic acid which is complementary to a target nucleic acid. An antisense molecule according to the invention is capable of interacting with, more specifically hybridizing with the target nucleic acid. By formation of the hybrid, transcription of the target gene(s) and/or translation of the target mRNA is reduced or blocked. Standard methods relating to antisense technology have been described (see, e.g., Melani et al., Cancer Res. (1991) 51:2897-2901).

A small molecule may be, for example, an organic molecule. Organic molecules relate or belong to the class of chemical compounds having a carbon basis, the carbon atoms linked together by carbon-carbon bonds. The original definition of the term organic related to the source of chemical compounds, with organic compounds being those carbon-containing compounds obtained from plant or animal or microbial sources, whereas inorganic compounds were obtained from mineral sources. Organic compounds can be natural or synthetic. Alternatively the compound may be an inorganic compound. Inorganic compounds are derived from mineral sources and include all compounds without carbon atoms (except carbon dioxide, carbon monoxide and carbonates). Preferably, the small molecule has a molecular weight of less than about 2000 amu, or less than about 1000 amu such as 500 amu, and even less than about 250 amu. The size of a small molecule can be determined by methods well-known in the art, e.g., mass spectrometry. The small molecules may be designed, for example, based on the crystal structure of STIM1 where sites presumably responsible for the biological activity, can be identified and verified in in vivo assays such as in vivo HTS assays.

All other inhibitors may also be identified and/or their function verified in HTS assays. High-throughput assays, independently of being biochemical, cellular or other assays, generally may be performed in wells of microtiter plates, wherein each plate may contain 96, 384 or 1536 wells. Handling of the plates, including incubation at temperatures other than ambient temperature, and bringing into contact of test compounds with the assay mixture is preferably effected by one or more computer-controlled robotic systems including pipetting devices. In case large libraries of test compounds are to be screened and/or screening is to be effected within short time, mixtures of, for example 10, 20, 30, 40, 50 or 100 test compounds may be added to each well. In case a well exhibits biological activity, said mixture of test compounds may be de-convoluted to identify the one or more test compounds in said mixture giving rise to said activity.

The determination of binding of potential inhibitors can be effected in, e.g., any binding assay, preferably biophysical binding assay, which may be used to identify binding test molecules prior to performing the functional/activity assay with the inhibitor. Suitable biophysical binding assays are known in the art and comprise fluorescence polarization (FP) assay, fluorescence resonance energy transfer (FRET) assay and surface plasmon resonance (SPR) assay.

In a further alternative method of identifying inhibitors, it is tested for inhibition of function in a cell transfected with said polynucleotide encoding the inhibitor if the inhibitor is of proteinaceous nature. This embodiment relates to a cellular screen. In a cellular screen inhibitors may be identified which exert their inhibitory activity by physically interacting with the target molecule, or alternatively (or additionally) by functionally interacting with said target molecule, i.e., by interfering with the pathway(s) present in the cells employed in the cellular assay.

In a more preferred embodiment of the pharmaceutical composition or the inhibitor of the invention, the activator of STIM1 is irreversibly inhibited by chemical modification or intracellular degradation. The preferred inhibitors of an activator of STIM1 include inhibitors of IP3-dependent Ca²⁺ store-release, inhibitors of SOC channel activation or inhibitors of STIM1 associated proteins involved in intracellular motility of STIM1.

The pharmaceutical composition of the invention may further comprise in the same or a separate container an antagonist of G-protein coupled receptors or signaling pathways, such as P2Y₁ inhibitors, P2Y₁₂ inhibitors, aspirin, inhibitors of PAR receptors.

The inhibitor of the invention, preferably in the pharmaceutical composition may have admixed thereto other coagulants which are known in the art, described, for example, in W02006/066878 which is specifically incorporated herein in its entirety.

The inhibitor of the invention may have admixed thereto or associated in a separate container an antagonist of G-protein coupled receptors or signalling pathways.

In a further preferred embodiment, the pharmaceutical composition or the inhibitor of the invention, the antagonist of G-protein coupled receptors or signaling pathways is an inhibitor of PAR receptors.

In another preferred embodiment of the inhibitor of the invention, the disorder related to venous or arterial thrombus formation is myocardial infarction, stroke, inflammation, complement activation, fibrinolysis, angiogenesis and/or diseases related to FXII-induced kinin formation such as hereditary angioedema, bacterial infection of the lung, trypanosome infection, hypotensitive shock, pancreatitis, chagas disease, thrombocytopenia or articular gout.

A myocardial infarction as used in the present invention relates to medical condition generally referred to as "heart attack" and is characterized by interrupted blood supply to the heart. The resulting ischemia (oxygen shortage) causes cellular damage and - depending on the length of ischemia - even tissue necrosis. Most commonly, myocardial infarct is due to the rupture of a vulnerable plaque that leads to a blockade of a vein or artery.

The term "stroke" is well-known in the art and sometimes also referred to as cerebrovascular accident (CVA). A stroke is a medical condition that is medically defined by reduced blood supply to the brain resulting in loss of brain function, inter alia due to ischemia. Said reduction in blood supply can be caused, for example, by thrombosis or embolism, or due to hemorrhage. Hence, strokes are generally classified into two major categories, i.e., i) ischemic and ii) hemorrhagic strokes. Ischemia is due to an interruption in blood circulation and hemorrhage is due to a rupture of a blood vessel, both scenarios ultimately leading to a reduced blood supply of the brain. The prevalent form of stroke is the ischemic stroke accounting for about 80% of strokes. In an ischemic stroke, blood supply to part of the brain is decreased, leading to dysfunction and necrosis of the brain tissue in that area. There are mainly four causative reasons: thrombosis (obstruction of a blood vessel by a blood clot forming locally), embolism (idem due to a blood clot from elsewhere in the body), systemic hypoperfusion (general decrease in blood supply, e.g. in shock) and venous thrombosis.

Despite the importance of ischemic stroke as the third leading cause of death and disability in industrialized countries treatment options in acute stroke are limited(22). Numerous attempts to attenuate infarct progression in acute stroke patients by conventional platelet aggregation inhibitors or anti-coagulation failed due to an excess of intracerebral hemorrhages(33). In accordance with the present invention, it was found that *STIM1*^{*-*/*-*} chimeras are protected from neuronal damage following transient cerebral ischemia without displaying an increased risk of intracranial hemorrhage (Fig.5).

In a particularly preferred embodiment of the inhibitor of the invention, said stroke is therefore ischemic stroke.

Further the invention relates to a combined pharmaceutical composition of an inhibitor of stromal interaction molecule 1 (STIM1) or an inhibitor of an activator of STIM1 and an antagonist of G-protein coupled receptors or signaling pathways for the simultaneous, separate or sequential use in therapy. This combined pharmaceutical composition can and optionally contain a pharmaceutically active carrier, excipient and/or diluent.

In a preferred embodiment the use in therapy for this combined pharmaceutical composition is the use in treating or preventing a disorder related to venous or arterial thrombus formation or as a lead compound for developing a drug for treating or preventing a disorder related to venous or arterial thrombus formation.

The Figures show:

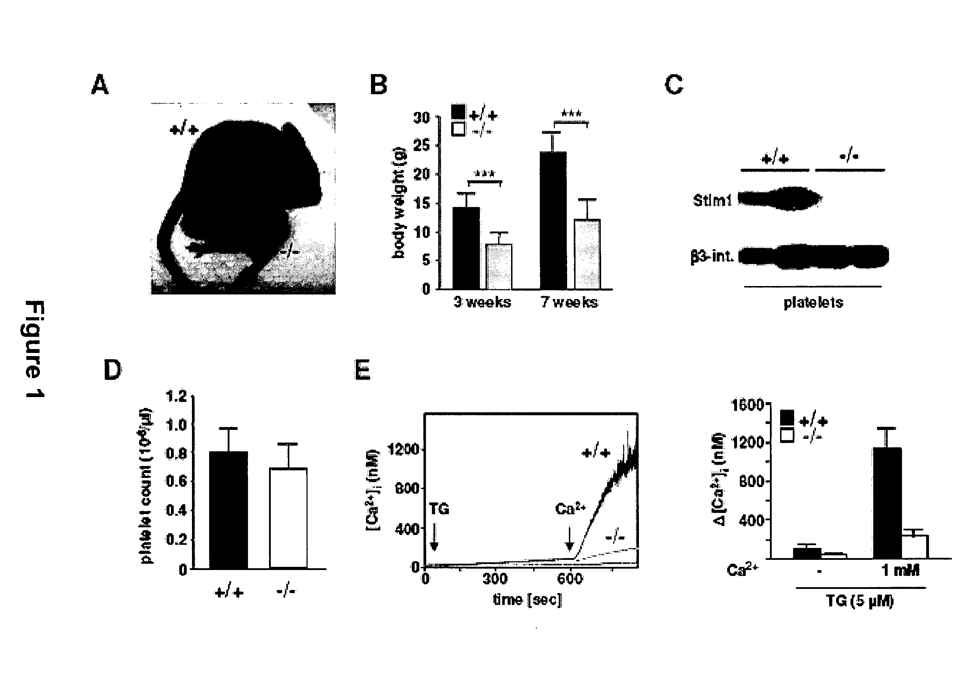

**Figure 1**. Defective SOCE in STIM1-deficient platelets. ***a***, Wild-type and *STIM1*^{*-*/*-*} littermates, 5 weeks old. ***b***, Body weights of wild-type (+/+) and *STIM1*^{*-*/*-*} (-/-) mice. ***c***, Western-blot analyses of platelet lysates. STIM1 was assessed using an antibody that can recognize the N-terminal region of the protein(14) (BD Transduction). An antibody to b3-integrin served as control. ***d***, Peripheral platelet counts in wild-type and *STIM1*^{*-*/*-*} mice. ***e***, Fura-2-loaded platelets were stimulated with 5 µM TG for 10 min followed by addition of extracellular Ca²⁺, and monitoring of [Ca²⁺]ᵢ. Representative measurements (left) and maximal Δ[Ca ²⁺]ᵢ ± SD (n = 4 per group) before and after addition of 1 mM Ca²⁺ (right) are shown.

**Figure 2****.** Defective agonist-induced Ca²⁺-signaling and aggregate formation under flow in *STIM1*^{*-*/*-*} platelets. Fura-2-loaded wild-type (black line) or *STIM1*^{*-*/*-*} (grey line) platelets were stimulated with thrombin (0.1 U/ml), ADP (10 µM) or CRP (10 µg/ml) in the presence of extracellular EGTA (1 mM) or Ca²⁺ (0.5 mM), and [Ca²⁺]_{¡} was monitored. Representative measurements a, and maximal Δ[Ca ²⁺]ᵢ ± SD (n = 4 per group) b, are shown. ***c***, Impaired aggregation of *STIM1*^{*-*/*-*} platelets (grey lines) in response to CRP and collagen, but not ADP and thrombin. ***d***, Flow cytometric analysis of allbb3 integrin activation and degranulation-dependent P-selectin exposure in response to thrombin (0.1 U/ml), ADP (10 µM), CRP (10 µg/ml) and CVX (1 µg/ml). Results are means ± SD of 6 mice per group. ***e***, *STIM1*^{*-*/*-*} platelets in whole blood fail to form stable thrombi when perfused over a collagen-coated (0.2 mg/ml) surface at a shear rate of 1,700 s⁻¹. Upper: representative phase contrast images. Lower: Mean surface coverage (left) and relative platelet deposition per mm² (right) ± SD (n=4).

**Figure 3****.** *In vivo* analysis of thrombosis and hemostasis. ***a-c,*** Mesenteric arterioles were treated with FeCl₃, and adhesion and thrombus formation of fluorescently-labeled platelets was monitored by *in vivo* video microscopy. Representative images (**a**), time to appearance of first thrombus > 20 µm (***b***), and time to vessel occlusion (c) are shown. Each symbol represents one individual. ***d***, ***e,*** The abdominal aorta was mechanically injured and blood flow monitored for 30 min or until complete occlusion occurred (blood flow stopped > 1 min). ***d***, Representative cross-sections of the abdominal aorta of mice with wild-type or *STIM1*^{*-*/*-*} platelets 30 min after injury. ***e***, Time to vessel occlusion. Each symbol represents one individual. ***f***, Tail bleeding times in wild-type and *STIM1*^{*-*/*-*} chimeras. Each symbol represents one individual.

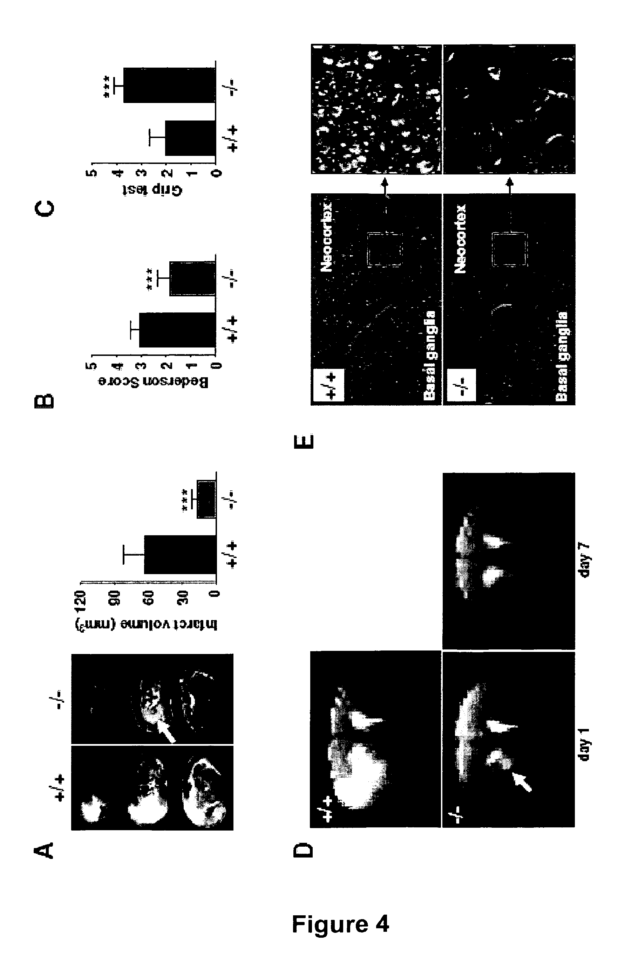

**Figure 4**. *STIM1^{-l-}* chimeras are protected from cerebral ischemia. ***a***, Left panel: Representative images of three corresponding coronal sections from control and *STIM1*^{*-*/*-*} chimeras mice stained with TCC 24 hrs after tMCAO. Infarcts in *STIM1^{-l-}*chimeras are restricted to the basal ganglia (white arrow). Right panel: Brain infarct volumes in control (n=7) and *STIM1*^{*-*/*-*} chimeras (n=7), *** p < 0.0001. ***b, c,*** Neurological Bederson score and grip test assessed at day 1 following tMACO of control (n=7) and *STIM1*^{*-*/*-*} chimeras animals (n=7), *** p < 0.0001. ***d***, The coronal T2-w MR brain image shows a large hyperintense ischemic lesion at day 1 after tMCAO in controls (left). Infarcts are smaller in *STIM1*^{*-*/*-*} chimeras (middle, white arrow), and T2-hyperintensity decreases by day 7 due to a "fogging" effect during infarct maturation (right). Importantly, hypointense areas indicating intracerebral hemorrhage were not seen in *STIM1*^{*-*/*-*} chimeras, demonstrating that STIM1 deficiency does not increase the risk of hemorrhagic transformation, even at advanced stages of infarct development. ***e***, Hematoxylin and eosin stained sections of corresponding territories in the ischemic hemispheres of control and *STIM1*^{*-*/-}chimeras. Infarcts are restricted to the basal ganglia in *STIM1*^{*-*/*-*} chimeras but consistently include the cortex in controls. Magnification x 100-fold.

The examples illustrate the invention.

### Example 1: Generation of STIM1-deficient mice

To address the function of STIM1 *in vivo,* the *STIM1* gene was disrupted in mice by insertion of an intronic gene-trap cassette. Mice heterozygous for the STIM1-null mutation were normal, while a majority (-70%) of mice lacking STIM1 (*STIM1*^{*-*/-}died within a few hours of birth. Marked cyanosis was noted before death, suggesting a cardio-pulmonary defect. Surviving *STIM1*^{*-*/*-*} mice exhibited marked growth retardation, achieving ∼50% of the weight of wild-type littermates at 3 and 7 weeks of age (Fig. 1a, b). Western blot analyses confirmed the absence of STIM1 in platelets (Fig. 1 c) and other tissues. Blood platelet counts (Fig. 1 d), mean platelet volume (MPV), and platelet surface receptors (Table 1) were normal, indicating that STIM1 is not essential for megakaryopoiesis or platelet production. Similarly, no differences were found in red blood cell counts, hematocrit, or the activated partial thromboplastin time (aPTT), a method for assessment of plasma coagulation (Table 2). To determine if STIM1 has a role in platelet SOCE, we induced SOC influx in wild-type and *STIM1*^{*-*/*-*} platelets with the SERCA (sarcoplasmic/endoplasmatic reticulum Ca²⁺ ATPase) pump inhibitor thapsigargin (TG). Interestingly, TG-induced Ca²⁺ store release was reduced ∼60% in *STIM1*^{*-*/*-*} platelets compared to wild-type controls (Fig. 1e). Furthermore, subsequent TG-dependent SOC influx was almost completely absent in *STIM1*^{*-*/*-*} cells (Fig. 1e). This demonstrates for the first time that STIM1 is essential for SOCE in platelets, and suggests that STIM1-dependent processes contribute to regulation of Ca²⁺ store content in these cells.

### Example 2: Defective SOC influx in STIM1^{-/-} platelets

Due to the early mortality and pronounced growth retardation in *STIM1*^{*-*/*-*} mice, all subsequent studies were performed with lethally irradiated wild-type mice transplanted with *STIM1*^{*-*/*-*} or wild-type bone marrow. Four weeks after transplantation, platelet counts were normal and STIM1-deficiency in platelets was confirmed by Western blot. To determine the significance of defective SOCE for agonist-induced platelet activation, we assessed changes in [Ca²⁺]ᵢ in response to ADP, thrombin, a *collagen related peptide* (CRP) that stimulates the collagen receptor glycoprotein (GP)VI (Fig. 2a, b), and the TxA₂ analogue U46619 (not shown). Ca²⁺ release from intracellular stores was reduced in *STIM1*^{*-*/*-*} platelets compared to control for all agonists, indicating reduced Ca²⁺ levels in stores in *STIM1*^{*-*/*-*} cells. In the presence of extracellular calcium, Ca²⁺ influx was dramatically reduced in *STIM1*^{*-*/*-*} platelets. Thus, STIM1-dependent SOCE is a crucial component of the Ca²⁺ signaling mechanism in platelets for all major agonists, and non-SOCE makes only a minor contribution, at least under the conditions tested.

### Example 3: STIM1 in platelet activation and thrombus formation

To test the functional consequences of this defect, we performed *ex vivo* aggregation studies. *STIM1*^{*-*/*-*} platelets aggregated normally to the G-protein coupled agonists ADP, thrombin (Fig. 2c) and U46619 (not shown), but responses to collagen and CRP (Fig. 2c) and the strong GPVI agonist convulxin (CVX) were significantly diminished. The activation defect was confirmed by flow cytometric analysis of integrin αllbβ3 activation, using the JON/A-PE antibody (Bergmeier, W. et al., 2002,. Cytometry 48:80-86) and of degranulation-dependent P-selectin surface exposure (Fig. 2d). Therefore, loss of STIM1-depbndent SOCE impairs GPVI-induced integrin activation and degranulation, whereas G-protein coupled agonists are still able to induce normal activation in *STIM1*^{*-*/*-*} platelets in these assays, despite the defect in [Ca²⁺]ᵢ signaling.

*In vivo,* platelet activation on ECM or a growing thrombus occurs in flowing blood, where locally produced soluble mediators are rapidly cleared. Under these conditions, reduced potency of platelet activators may become limiting, particularly at the high flow rates found in arteries and arterioles. Therefore, we analyzed the ability of *STIM1*^{*-*/*-*} platelets to form thrombi on collagen-coated surfaces in a whole blood perfusion system (Nieswandt, B. et al., 2001, EMBO J 20:2120-2130). Under high shear conditions (1,700 s⁻¹), wild-type platelets adhered to collagen fibers and formed aggregates within 2 min that consistently grew into large thrombi by the end of the perfusion period (Fig. 2e). In sharp contrast, *STIM1*^{*-*/*-*} platelets exhibited reduced adhesion, and three-dimensional growth of thrombi was markedly impaired. As a consequence, the surface area covered by platelets and the total thrombus volume were reduced by ∼42% and ∼81%, respectively. Similar results were obtained at intermediate shear rates (1,000 s⁻¹ - data not shown). These findings indicate that STIM1-mediated SOCE is required for efficient platelet activation on collagen, and on the surface of growing thrombi under conditions of high shear.

### Example 4: Unstable arterial thrombi in STIM1^{-/-} mice

As platelet aggregation may contribute to pathologic occlusive thrombus formation, we studied the effects of STIM1-deficiency on ischemia and infarction by *in vivo* fluorescence microscopy following ferric chloride-induced mesenteric arteriole injury. In all wild-type chimeras, the formation of small aggregates was observed -5 minutes after injury, with progression to complete vessel occlusion in 8 of 10 mice within 30 min (mean occlusion time: 16.5 ± 2.8 min) (Fig. 3b, c). In contrast, aggregate formation was significantly delayed in ∼50% of the *STIM1*^{*-*/*-*} chimeras, and formation of stable thrombi was almost completely abrogated. This defect was due to the release of individual platelets from the surface of the thrombi, and not to embolization of large thrombus fragments. Blood flow was maintained throughout the observation period in 9 of 10 vessels, demonstrating a crucial role for STIM1 during occlusive thrombus formation. This was confirmed in a second arterial thrombosis model, where the abdominal aorta was mechanically injured and blood flow was monitored with an ultrasonic flow probe. While 10 of 11 control chimeras formed irreversible occlusions within 16 minutes (mean occlusion time: 4.4 ± 4.1 min), occlusive thrombus formation did not occur in 6 of 8 *STIM1*^{*-*/*-*} chimeras during the 30 min observation period (Fig. 3d, e). These results demonstrate that STIM1 is required for the propagation and stabilization of platelet-rich thrombi in small and large arteries, irrespective of the type of injury.

To test whether the defect in *STIM1*^{*-*/*-*} platelets impaired hemostasis, we measured tail bleeding times. While bleeding stopped in 28 of 30 control mice within 10 min (mean: 6.6 ± 2.4 min), bleeding was highly variable in *STIM1*^{*-*/*-*} chimeras, with 5 of 31 (20%) mice bleeding for >20 minutes (Fig. 3f). These results show that STIM1 is required for normal hemostasis.

### Example 5: STIM1 is an essential mediator of ischemic brain infarction

Ischemic stroke is the third leading cause of death and disability in industrialized countries (Murray, C.J. and Lopez,A.D., 1997, Lancet 349:1269-1276). Although it is well established that microvascular integrity is disturbed during cerebral ischemia (Zhang, Z.G. et al., 2001, Brain Res. 912:181-194), the signaling cascades involved in intravascular thrombus formation in the brain are poorly understood. To determine the importance of STIM1-dependent SOCE in this process, we studied the development of neuronal damage in *STIM1*^{*-*/*-*} chimeras following transient cerebral ischemia in a model that depends on thrombus formation in microvessels downstream from a middle cerebral artery (MCA) occlusion (Choudhri, T.F. et al., 1998, J Clin Invest 102:1301-1310; del Zoppo, G.J. and Mabuchi,T., 2003, J. Cereb. Blood Flow Metab 23:879-894). To initiate transient cerebral ischemia, a thread was advanced through the carotid artery into the MCA and allowed to remain for one hour (transient MCA occlusion - tMCAO), reducing regional cerebral flow by >90%. In *STIM1*^{*-*/*-*} chimeras, infarct volumes 24 hours after reperfusion, as assessed by TTC staining, were reduced to < 30% of the infarct volumes in control chimeras (17.0 ± 4.4 mm³ versus 62.9 ± 19.3 mm³, p < 0.0001) (Fig. 4a). Reductions in infarct size were functionally relevant, as the Bederson score assessing global neurological function (1.86 ± 0.48 versus 3.07 ± 0.35, respectively; p < 0.0001) and the grip test, which specifically measures motor function and coordination (3.71 ± 0.39 versus 2.00 ± 0.65, respectively; p < 0.0001), were significantly better in *STIM1*^{*-*/*-*} chimeras compared to controls (Fig. 4b, c). Serial magnetic resonance imaging (MRI) on living mice was used to confirm the protective effect of STIM1-deficiency on infarct development. Hyperintense ischemic infarcts on T2-w MRI in *STIM1*^{*-*/*-*} chimeras were <10% of the size of infarcts in control chimeras 24 hrs after tMCAO (p < 0.0001, Fig. 4d). Importantly, infarct volume did not increase between day 1 and day 7, indicating a sustained protective effect for STIM1-deficiency. Moreover, no intracranial hemorrhage was detected on T2-weighted gradient echo images, a highly sensitive MRI sequence for detection of blood (Fig. 4d), indicating that STIM1-deficiency in hematopoietic cells is not associated with an increase in bleeding complications in the brain. Consistent with the TTC stains and MRI images, histological analysis revealed massive ischemic infarction of the basal ganglia and neocortex in control chimeras, but only limited infarction of the basal ganglia in *STIM1*^{*-*/*-*} chimeras (Fig. 4e). The density of CD3-positive T cell and monocyte/macrophage infiltrates in brain infarcts was low, and did not differ between *STIM1*^{*-*/*-*} and control chimeras at 24h.

### Example 6: MATERIALS AND METHODS

**Mice**. Animal studies were approved by the Bezirksregierung of Unterfranken. Generation of *STIM1*^{*-*/*-*} mice was done as follows. A mouse ES cell line (RRS558) containing an insertional disruption in the *STIM1* gene was obtained from BayGenomics. The identity of the trapped gene as *STIM1* was confirmed by RT-PCR and Southern Blot analysis. Male chimeras from this ES cell line were bred to C57BI/6 females to generate *STIM1*^{*-*/*-*} mice, which were intercrossed to produce *STIM1*^{*-*/*-*} mice. *Generation of bone marrow chimeras.* 5-6 weeks old C57BI/6 female mice were lethally irradiated with a single dose of 10 Gy, and bone marrow cells from 6 weeks old wild type or *STIM1*^{*-*/*-*} mice were injected intravenously into the irradiated mice (4 x 10⁶ cells/mouse). Four weeks after transplant, platelet counts were determined and STIM1-deficiency confirmed by Western Blot. All recipient animals received acidified water containing 2g/l Neomycin sulphate for 6 weeks after transplantation.

**Chemicals and antibodies**. Anesthetic drugs: medetomidine (Pfizer, Karlsruhe, Germany), midazolam (Roche Pharma AG, Grenzach-Wyhlen, Germany), fentanyl (Janssen-Cilag GmbH, Neuss, Germany) and antagonists: atipamezol (Pfizer, Karlsruhe, Germany), flumazenil and naloxon (both from Delta Select GmbH, Dreieich, Germany) were used according to the regulation of the local authorities. ADP (Sigma, Deisenhofen, Germany), U46619 (Alexis Biochemicals, San Diego, USA), thrombin (Roche Diagnostics, Mannheim, Germany), collagen (Kollagenreagent Horm, Nycomed, Munich, Germany) and thapsigargin (Molecular Probes) were purchased. Monoclonal antibodies conjugated to fluorescein isothiocyanate (FITC) or phycoerythrin (PE), or DyLight-488 were from Emfret Analytics (Würzburg, Germany). Anti-STIM1 antibodies were from BD Transduction and Abnova.

**Intracellular calcium measurements. Platelet intracellular calcium measurements** were performed as described (Heemskerk, J.W. et al, 1991, Lett. 284:223-226). Briefly, platelets isolated from blood were washed, suspended in Tyrode's buffer without calcium, and loaded with fura-2/AM (5 µM) in the presence of Pluronic F-127 (0.2 µg/ml) (Molecular Probes) for 30 min at 37°C. After labeling, platelets were washed once and resuspended in Tyrode's buffer containing 0.5 mM Ca²⁺ or 1 mM EGTA. Stirred platelets were activated with agonists, and fluorescence was measured with a PerkinElmer LS 55 fluorimeter. Excitation was alternated between 340 and 380 nm, and emission was measured at 509 nm. Each measurement was calibrated using Triton X-100 and EGTA.

**Platelet aggregometry**. Changes in light transmission of a suspension of washed platelets (200 µl with 0.5 x 10⁶ platelets/µl) was measured in the presence of 70 µg/ml human fibrinogen. Transmission was recorded on a Fibrintimer 4 channel aggregometer (APACT Laborgeräte und Analysensysteme, Hamburg, Germany) over ten minutes, and was expressed in arbitrary units with buffer representing 100% transmission.

**Flow cytometry**. Heparinized whole blood was diluted 1:20 with modified Tyrode-HEPES buffer (134 mM NaCl, 0.34 mM Na₂HPO₄, 2.9 mM KCI, 12 mM NaHCO₃, 20 mM HEPES [*N*-2-hydroxyethylpiperazine-*N*'-2-ethanesulfonic acid], pH 7.0) containing 5 mM glucose, 0.35% bovine serum albumin (BSA), and 1 mM CaCl₂. For glycoprotein expression and platelet count, blood samples were incubated with appropriate fluorophore-conjugated monoclonal antibodies for 15 min at RT and analyzed on a FACScalibur instrument (Becton Dickinson, Heidelberg, Germany). For activation studies, blood samples were washed twice with modified Tyrode-HEPES buffer, incubated with agonist for 15 minutes, stained with fluorophore-labeled antibodies for 15 minutes at RT, and then analyzed.

**Adhesion under flow conditions**. Rectangular coverslips (24 x 60 mm) were coated with 0.2 mg/ml fibrillar type I collagen (Nycomed, Munich, Germany) for 1 h at 37°C and blocked with 1% BSA. Heparinized whole blood was labeled with a Dylight-488 conjugated anti-GPIX Ig derivative at (0.2 µg/ml) and perfusion was performed as described(21). Briefly, transparent flow chambers with a slit depth of 50 µm and equipped with collagen-coated coverslips were rinsed with Hepes buffer and connected to a syringe filled with anti-coagulated blood. Perfusion was carried out at RT using a pulse-free pump at medium (1000 s⁻¹) or high (1700 s⁻¹) shear rates. During perfusion, microscopic phase-contrast images were recorded in real-time. The chambers were rinsed by a 10 min perfusion with Hepes buffer pH 7.45 at the same shear, and phase-contrast and fluorescent pictures were recorded from at least five different microscopic fields (40 x objectives). Image analysis was performed off-line using Metavue software (Visitron, Munich, Germany). Thrombus formation was expressed as the mean percentage of total area covered by thrombi, and as the mean integrated fluorescence intensity per mm².

Bleeding time. Mice were anesthetized and a 3 mm segment of the tail tip was removed with a scalpel. Tail bleeding was monitored by gently absorbing blood with filter paper at 20 second intervals, without making contact with the wound site. When no blood was observed on the paper, bleeding was determined to have ceased. Experiments were stopped after 20 minutes.

**Intravital microscopy of thrombus formation in FeCl₃ injured mesenteric arterioles**. Four weeks after bone marrow transplantations, chimeras were anesthetized, and the mesentery was exteriorized through a midline abdominal incision. Arterioles (35-60µm diameter) were visualized with a Zeiss Axiovert 200 inverted microscope (x10) equipped with a 100-W HBO fluorescent lamp source, a HBO fluorescent lamp source, and a CooISNAP-EZ camera (Visitron, Munich Germany). Digital images were recorded and analyzed off-line using Metavue software. Injury was induced by topical application of a 3 mm² filter paper saturated with FeCl₃ (20%) for 10 sec. Adhesion and aggregation of fluorescently labeled platelets (Dylight-488 conjugated anti-GPIX Ig derivative) in arterioles was monitored for 30 min or until complete occlusion occurred (blood flow stopped for > 1 min).

**Aorta occlusion model.** A longitudinal incision was used to open the abdominal cavity of anesthetized mice and expose the abdominal aorta. An ultrasonic flow probe was placed around the vessel and thrombosis was induced by a single firm compression with a forceps. Blood flow was monitored until complete occlusion occurred; or 30 minutes had elapsed.

**Murine stroke model**. Experiments were conducted on 10-12 wk-old *STIM1*^{*-*/*-*} or control chimeras according to published recommendations for research in mechanism-driven basic stroke studies (Dirnagl, U., 2006, J. Cereb. Blood Flow Metab 26:1465-1478). Transient middle cerebral artery occlusion (tMCAO) was induced under inhalation anesthesia using the intraluminal filament (6021 PK10; Doccol Company) technique (Kleinschnitz, C. et al., 2007, Circulation 115:2323-2330). After 60 min, the filament was withdrawn to allow reperfusion. For measurements of ischemic brain volume, animals were sacrificed 24 h after induction of tMCAO and brain sections were stained with 2% 2,3,5-triphenyltetrazolium chloride (TTC; Sigma-Aldrich, Germany). Brain infarct volumes were calculated and corrected for edema as described (Kleinschnitz, C. et al., 2007, Circulation 115:2323-2330).

**Neurological testing**. Neurological function was assessed by two independent and blinded investigators 24 h after tMACO. Global neurological status was scored according to Bederson et al. (Bederson, J.B. et al., 1986, Stroke 17:472-476). Motor function was graded using the grip test (Moran, P.M. et al., 1995, Proc. Natl. Acad. Sci. U. S. A 92:5341-5345).

**Stroke assessment by MRI**. MRI was performed 24 h and 7 d after stroke on a 1.5 T unit (Vision; Siemens) under inhalation anesthesia. A custom made dual channel surface coil was used for all measurements (A063HACG; Rapid Biomedical). The MR protocol included a coronal T2-w sequence (slice thickness 2 mm), and a coronal T2-w gradient echo CISS sequence (Constructed Interference in Steady State; slice thickness1 mm). MR images were transferred to an external workstation (Leonardo; Siemens) for data processing. Visual analysis of infarct morphology and ICH was performed in a blinded manner. Infarct volumes were calculated by planimetry of hyperintense areas on high-resolution CISS images.

**Histology**. Formalin-fixed brains embedded in paraffin (Histolab Products AB) were cut into 4-µm thick sections and mounted. After removal of paraffin, tissues were stained with hematoxylin and eosin (Sigma-Aldrich).

**Statistics**. Results from at least three experiments per group are presented as mean ± SD. Differences between wild-type and *STIM1*^{*-*/*-*} groups were assessed by 2-tailed Student's *t* test. Stroke model: Results are presented as mean ± SD. Infarct volumes and functional data were tested for Gaussian distribution with the D'Agostino and Pearson omnibus normality test and then analyzed using the two-tailed student's t-test. For statistical analysis, PrismGraph 4.0 software (GraphPad Software, USA) was used. P-values < 0.05 were considered statistically significant.

**Table 1. Platelet membrane glycoprotein expression in STIM1^{-/-} platelets. Diluted whole blood was stained with fluorophore-labeled antibodies at saturating concentrations for 15 min at RT and analyzed on a FACScalibur (Becton Dickinson, Heidelberg). Platelets were gated by FSC/SSC characteristics. Results are given as the mean fluorescence intensity ± SD of 6-12 mice per group. Mean platelet volume (MPV) was determined on a Sysmex cell counter and is expressed as mean ± SD of 6 mice per group.**

| | ***STIM1*^{+/+}** | ***STIM1*^{*-*/*-*}** |
|---|---|---|
| **GPlb** | 388 ± 46 | 412 ± 25 |
| **GPV** | 253 ± 26 | 274 ± 34 |
| **GPIX** | 391 ± 38 | 381 ± 26 |
| **GPVI** | 50 ± 6 | 46±12 |
| **α2** | 111 ± 6 | 115 ± 11 |
| **β1** | 163±12 | 161 ± 11 |
| **αllbβ3** | 694 ± 59 | 722 ± 66 |
| **CD9** | 1490 ± 72 | 1495 ± 195 |
| **MPV (fl)** | 5.5 ± 0.2 | 5.4 ± 0.3 |

**Table 2. Hematology and hemostasis in STIM1^{-/-} chimeras. Erythrocyte counts per nl and coagulation parameters for control and STIM1^{-/-} chimeras. The abbreviations are hematocrit (HCT), activated partial thromboplastin time (aPTT), prothrombin time (PT), and thrombin clotting time (TCT). Values given are mean values ± SD of 5 mice for each genotype.**

| | ***STIM1*^{+/+}** | ***STIM1*^{-/-}** |
|---|---|---|
| **Erythrocytes** | 8450 ± 139 | 8250 ± 264 |
| **HCT [%]** | 40.8 ± 0.4 | 41.7 ± 1.9 |
| **aPTT [sec]** | 37.7 ± 5.1 | 38.7 ± 3.1 |
| **PT [sec]** | 9.4 ± 0.5 | 9.8 ± 0.7 |
| **TCT [sec]** | 19.2 ± 2.6 | 21.8 ± 1.0 |
| **Fibrinogen** | 2.2 ± 0.1 | 2.8 ± 0.6 |

## Claims

1. A pharmaceutical composition comprising an inhibitor of stromal interaction molecule 1 (STIM1) or an inhibitor of an activator of STIM1 and optionally a pharmaceutically active carrier, excipient and/or diluent.

2. The pharmaceutical composition of claim 1 wherein the inhibitor is an antibody, an aptamer, an siRNA, an shRNA, or a further molecule effecting RNAi, a ribozyme, an antisense nucleic acid molecule or a small molecule.

3. The pharmaceutical composition of claim 1 or 2 wherein the inhibitor of an activator of STIM1 is an inhibitor of IP3 dependent Ca²⁺ store-release, SOC channel activation or a STIM1 associated protein involved in intracellular motility of STIM1.

4. The pharmaceutical composition of any one of claims 1 to 3 further comprising in the same or a separate container an antagonist of G-protein coupled receptors or signaling pathways.

5. The pharmaceutical composition of claim 4 wherein the antagonist of G-protein coupled receptors or signaling pathways is an inhibitor of P2Y₁, P2Y₁₂, PAR receptors, or aspirin.

6. An inhibitor of stromal interaction molecule 1 (STIM1) or an inhibitor of an activator of STIM1 for treating or preventing a disorder related to venous or arterial thrombus formation or as a lead compound for developing a drug for treating or preventing a disorder related to venous or arterial thrombus formation.

7. The inhibitor of claim 6 wherein the inhibitor is an antibody, an aptamer, an siRNA, an shRNA, or a further molecule effecting RNAi, a ribozyme, an antisense nucleic acid molecule or a small molecule.

8. The inhibitor of claim 6 or 7 wherein the inhibitor of an activator of STIM1 is an inhibitor of IP3 dependent Ca²⁺ store-release, SOC channel activation or a STIM1 associated protein involved in intracellular motility of STIM1.

9. The inhibitor of any one of claims 6 to 8 having admixed thereto or associated in a separate container an antagonist of G-protein coupled receptors or signaling pathways.

10. The inhibitor of claim 9 wherein the antagonist of G-protein coupled receptors or signaling pathways is an inhibitor of P2Y₁, P2Y₁₂, PAR receptors, or aspirin.

11. The inhibitor of any one of claims 6 to 10 wherein the disorder related to venous or arterial thrombus formation is myocardial infarction, stroke, inflammation, complement activation, fibrinolysis, angiogenesis and/or diseases related to FXII-induced kinin formation such as hereditary angioedema, bacterial infection of the lung, trypanosome infection, hypotensitive shock, pancreatitis, chagas disease, thrombocytopenia or articular gout.

12. The inhibitor of any one of claims 6 to 10 for treating myocardial infarction, stroke, inflammation, complement activation, fibrinolysis, angiogenesis and/or diseases related to FXII-induced kinin formation such as hereditary angioedema, bacterial infection of the lung, trypanosome infection, hypotensitive shock, pancreatitis, chagas disease, thrombocytopenia or articular gout.

13. The inhibitor of claim 11 or 12 wherein said stroke is ischemic stroke.

14. Use of an inhibitor of stromal interaction molecule 1 (STIM1) or an inhibitor of an activator of STIM1 for the manufacture of a medicament for treating or preventing a disorder related to venous or arterial thrombus formation or as a lead compound for developing a drug for treating or preventing a disorder related to venous or arterial thrombus formation.

15. A combined pharmaceutical composition of an inhibitor of stromal interaction molecule 1 (STIM1) or an inhibitor of an activator of STIM1 and an antagonist of G-protein coupled receptors or signaling pathways and optionally a pharmaceutically active carrier, excipient and/or diluent for the simultaneous, separate or sequential use in therapy.

16. The combined pharmaceutical composition of claim 15 for treating or preventing a disorder related to venous or arterial thrombus formation or as a lead compound for developing a drug for treating or preventing a disorder related to venous or arterial thrombus formation.
